# EUROPEAN PATENT APPLICATION

(11) **EP 2 476 377 A1**
(43) Date of publication of application: **18.07.2012**
(21) Application number: 12162214.6
(22) Date of filing: 18.02.2009
(51) Int. Cl.: A61B 10/02, A61B 19/00

(54) **Biopsy site marker applier**

(30) Priority: 19.02.2008 US 29685 P
(62) Divisional of application: 09250423.2
(71) Applicant: Devicor Medical Products, Inc., Pleasant Prairie, WI 53158 (US)
(72) Inventor: Parihar,, Shailendra K., Mason, OH Ohio 45040 (US); Ludzack,, Michael R., Mason, OH Ohio 45040 (US); Haberstitch,, Wells D., Loveland, OH Ohio 45140 (US)
(74) Representative: Tunstall, Christopher Stephen

(57) **Abstract**

A biopsy marker applier is described and illustrated. The biopsy marker applier includes a cannula and at least one marker disposed within the cannula. A member is positioned on the cannula, such as sliding member disposed to slide along the cannula. The sliding member can be used to control or limit the depth of insertion of the biopsy marker applier in a biopsy device and/or a patient.

## Description

### BACKGROUND

Biopsy samples have been obtained in a variety of ways in various medical procedures using a variety of devices. An exemplary biopsy device is the MAM1VIOTOME device from Ethicon Endo-Surgery, Inc. of Cincinnati, Ohio. Biopsy devices may be used under stereotactic guidance, ultrasound guidance, MRI guidance, or otherwise. Further exemplary biopsy devices are disclosed in U.S. Pat. No. 5,526,822, entitled "Method and Apparatus for Automated Biopsy and Collection of Soft Tissue," issued June 18, 1996; U.S. Pat. No. 6,086,544, entitled "Control Apparatus for an Automated Surgical Biopsy Device," issued July 11, 2000; U.S. Pub. No. 2003/0109803, entitled "MRI Compatible Surgical Biopsy Device," published June 12, 2003; U.S. Pub. No. 2007/0118048, entitled "Remote Thumbwheel for a Surgical Biopsy Device," published May 24, 2007; U.S. Provisional Patent Application Serial No. 60/869,736, entitled "Biopsy System," filed December 13, 2006; U.S. Provisional Patent Application Serial No. 60/874,792, entitled "Biopsy Sample Storage," filed December 13, 2006; and U.S. Non-Provisional Patent Application Serial No. 11/942,785, entitled "Revolving Tissue Sample Holder for Biopsy Device," filed November 21, 2007. The disclosure of each of the above-cited U.S. Patents, U.S. Patent Application Publications, U.S. Provisional Patent Applications, and U.S. Non-Provisional Patent Application is incorporated by reference herein.

In some settings, it may be desirable to mark the location of a biopsy site for future reference. For instance, one or more markers may be deposited at a biopsy site before, during, or after a tissue sample is taken from the biopsy site. Exemplary marker deployment tools include the MAMMOMARK, MICROMARK, and CORMARK devices from Ethicon Endo-Surgery, Inc. of Cincinnati, Ohio. Further exemplary devices and methods for marking a biopsy site are disclosed in U.S. Pub. No. 2005/0228311, entitled "Marker Device and Method of Deploying a Cavity Marker Using a Surgical Biopsy Device," published October 13, 2005; U.S. Pat. No. 6,996,433, entitled "Imageable Biopsy Site Marker," issued February 7, 2006; U.S. Pat. No. 6,993,375, entitled "Tissue Site Markers for In Vivo Imaging," issued January 31, 2006; U.S. Pat. No. 7,047,063, entitled "Tissue Site Markers for In Vivo Imaging," issued May 16, 2006; U.S. Pat. No. 7,229,417, entitled "Methods for Marking a Biopsy Site," issued June 12, 2007; U.S. Pat. No. 7,044,957, entitled "Devices for Defining and Marking Tissue," issued May 16, 2006; U.S. Pat. No. 6,228,055, entitled "Devices for Marking and Defining Particular Locations in Body Tissue," issued May 8, 2001; and U.S. Pat. No. 6,371,904, entitled "Subcutaneous Cavity Marking Device and Method," issued April 16, 2002. The disclosure of each of the above-cited U.S. Patents and U.S. Patent Application Publications is incorporated by reference herein.

SUMMARY

It may be desirable in some contexts to minimize the contact between a user's hand and a portion of a marker applier device that will be inserted in a patient. It may also be desirable to selectively adjust and/or limit the depth to which a marker applier device may be inserted through a biopsy device. In addition, it may also be desirable in some contexts to provide a clear indication relating to the depth at which a marker applier is inserted in a patient or biopsy device.

In one embodiment, the invention provides a biopsy site marker applier. In another embodiment, the invention provides a method for using a biopsy site marker applier.

For instance, and by way of non-limiting example, the present invention can include a marker applier having a movable member that can be positioned at two or more discrete or continuous position along a portion of the marker applier. For instance, the marker applier can include a sliding member disposed on a cannula such that the sliding member's position can be adjusted along the axial length of the cannula.

The marker applier can be provided in a package, such as a packaging tray, and the initial position of the sliding member on the cannula can be fixed by a recess or other feature of the tray. The sliding member can provide a gripping portion, such as a gripping surface, which allows the user to hold the marker applier without touching the portion of the cannula that will ultimately be inserted into a biopsy device. In those cases where the cannula is a relatively long, flexible elongate hollow member, the sliding member can grasped or supported by the users hand to steer or direct the tip of the marker applier into the biopsy device.

The marker applier may include a feature, such as a lock or latch that is operable to selectively lock and unlock the sliding member with respect to to the cannula, thereby allowing the sliding member to be positioned at discrete or continuous positions along the length of the cannula. The marker applier or the package in which the marker applier is provided may include one or more indicators, such as tactile and/or visual indicators. The indicators can be associated with, or can indicate, an insertion depth of the cannula within a biopsy device and/or a patient's body.

In one embodiment, the present invention can also provide a biopsy system. The biopsy system can include a biopsy device operable for taking one or more biopsy samples from a patient, and a biopsy marker applier including a movable member. The biopsy device can include a piercing member having a piercer lumen, a tissue receiving openings, and a cutter rotatable and translatable in the piercer lumen for severing tissue received within the tissue receiving opening of the piercing member. The biopsy marker applier can include a cannula having a marker lumen and at least one marker disposed in the marker lumen, and a movable member, such as a sliding member, positionable on the cannula; wherein the movable member is positionable on the cannula at a plurality of locations along the length of the cannula. The slidable member can adjustably positioned on the outer surface of the cannula to provide a desired depth of insertion of the cannula within the biopsy device or the patient.

In another embodiment, the invention also provides a method for using a marker applier. The can comprise the steps of obtaining a biopsy marker applier comprising at least one biopsy marker; determining a desired depth of insertion of a portion of the biopsy marker applier within a biopsy device or a patient; and positioning a movable member along a portion of the biopsy marker applier to a position corresponding to the desired depth of insertion. 16. The method of Claim 14 further comprising the step of inserting a portion of the biopsy marker applier into a biopsy device adapted to obtain a biopsy sample.

The step of positioning may comprises sliding the movable member distally with respect to a distal tip of the biopsy marker applier. The step of positioning may comprise sliding the movable member proximally with respect to a distal tip of the biopsy marker. The method may further comprise the step of sliding the movable member to a position designated by an indicator associated with a desired depth of insertion.

### BRIEF DESCRIPTION OF THE DRAWINGS

It is believed the present invention will be better understood from the following description of certain examples taken in conjunction with the accompanying drawings, in which like reference numerals identify the same elements and in which:

FIG. 1 depicts a perspective view of an exemplary marker deployment tool;

FIG. 2 depicts a partial cross-sectional view of the marker deployment tool of FIG. 1 inserted in an exemplary biopsy device, deploying a marker;

FIG. 3 depicts the marker deployment tool of FIG. 1, modified with an exemplary sliding member;

FIG. 4 depicts the modified marker deployment tool of FIG. 3 being removed from packaging;

FIG. 5 depicts the modified marker deployment tool of FIG. 3 being coupled with an exemplary biopsy device;

FIG. 6 depicts the modified marker deployment tool of FIG. 3 being adjusted relative to the biopsy device;

FIG. 7 depicts the marker deployment tool of FIG. 1, modified with another exemplary sliding member;

FIG. 8 depicts the modified marker deployment tool of FIG. 7 coupled with an exemplary biopsy device;

FIG. 9 depicts another exemplary sliding member

FIG. 10 depicts another exemplary sliding member; and

FIG. 11 depicts another exemplary sliding member.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

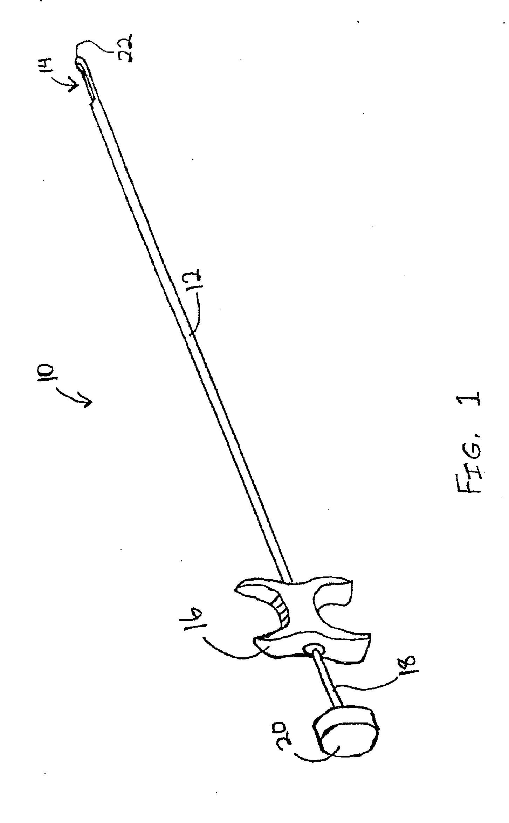

As shown in FIG. 1, an exemplary marker applier (10) includes an elongate outer cannula (12) having a transverse opening (14) formed near its distal end. The distal end of cannula (12) comprises a closed tip (22). A grip (16) is provided at the proximal end of cannula (12). Cannula (12) and tip (22) may be formed of PEBAX, any suitable polymer, metals, or any other suitable materials, including combinations thereof A push rod (18) is inserted coaxially in cannula (12) and is configured to translate within cannula (12). Rod (18) has sufficient rigidity to push a marker (30) out through opening (14) as will be described in greater detail below. However, rod (18) and cannula (12) are nevertheless flexible in this example. A plunger (20) is provided at the proximal end of rod (18) for forcing rod (18) distally in cannula (12). In particular, a user may grasp grip (16) with two fingers, and may push on plunger (20) using the thumb on the same hand, such that applier (10) may be operated by a user's single hand. A spring (not shown) or other feature may be provided about rod (18) to bias rod (18) proximally.

Marker applier (10) may be used to deploy a marker (30) via opening (14) within tissue, such as to mark a particular location within a patient. By way of example only, a marker (30) may mark the site of a biopsy. Of course, there may be a variety of other contexts for marking a location in a patient's body. In the present example, however, marker applier (10) is introduced to a biopsy site using the same instrument or biopsy device (100) that was used to collect a tissue sample from the biopsy site. Several such biopsy devices are disclosed in the various patents and patent applications that have been referred to and incorporated by reference herein, though any other biopsy devices may be used.

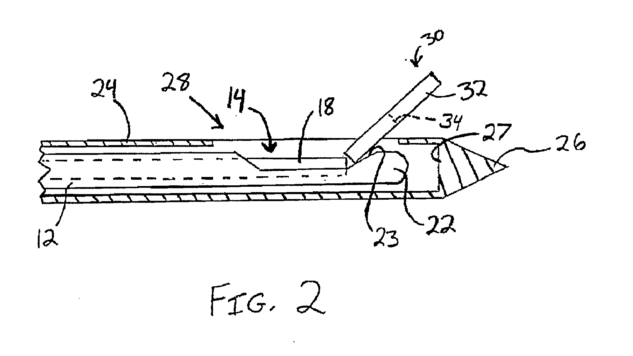

An exemplary use of a marker applier (10) is shown in FIG. 2. In particular, FIG. 2 shows the distal end of a marker applier (10) disposed within the outer needle (24) of a biopsy device (100). The outer needle (24) has a tissue piercing tip (26) and a transverse opening (28) proximal to the tip (26). By way of example only, biopsy device (100) may include any of the various biopsy devices disclosed in U.S. Non-Provisional Patent Application Serial No. 11/942,785, entitled "Revolving Tissue Sample Holder for Biopsy Device," filed November 21, 2007, the disclosure of which is incorporated by reference herein.

In the present example, needle (24) and applier (10) are configured such that opening (14) of cannula (12) and opening (28) of needle (24) will be substantially aligned when cannula (12) is inserted in needle (24). In other words, opening (14) of cannula (12) is positioned relative to opening (28) of needle (24) such that a marker (30) may be deployed through both openings (14, 28). To deploy marker (30), a user actuates plunger (20), driving rod (18) distally. Tip (22) of marker applier (10) includes a proximally facing ramped surface (23), which urges marker (30) outwardly through openings (14, 28) and into the biopsy site as rod (18) is driven distally. Of course, needle (24) and marker applier (10) may have a variety of other structures, configurations, and relationships at their distal ends (among other places), and may be used in a variety of alternative ways.

Marker (30) of the present example comprises a marker body (32) and a marking element (34). In some versions, marker body (32) is visible under ultrasound imaging, while marking element (34) is visible under MRI and X-ray, among other imaging modalities. For instance, marker body (32) may be formed of bovine collagen, cellulose, PLA/PGA, glycoprene, gelatinous materials such as hydrogel, and/or any other suitable material(s), including combinations thereof Furthermore, marker body (32) may be biodegradable or bioapsorbable, or may have other properties. Marking element (34) may comprise a titanium wire, pellet, or other structure, and may be MRI compatible or not MRI compatible. Of course, any other material(s) may be used for marking element (34), including combinations thereon In some versions, marker body (32) is formed of a square collagen pad that is folded and/or rolled about a titanium marking element (34) to form a substantially cylindraceous marker (30). Marker (30) is then compressed radially inward in this example before being inserted into cannula (12) to load marker applier (10) for deployment. In other versions, marker (30) is formed of a collagen dowel. Of course, marker (30) may have a variety of alternative configurations, may be formed using a variety of techniques and materials, and may be used in a variety of other ways.

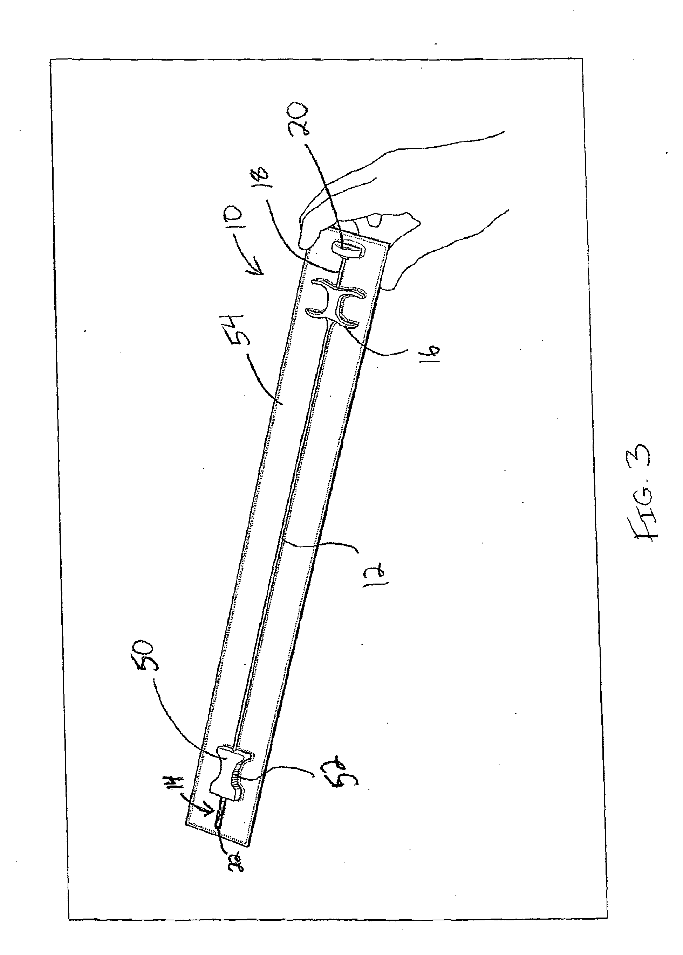

FIG. 3 shows one way in which marker applier (10) may be modified. In particular, a slider (50) is disposed about cannula (12) of marker applier (10). Slider (50) has a pair of concave gripping regions (52) along opposing sides, providing a generally bowtie-shaped appearance. Alternatively, slider (50) may have any other suitable appearance, such that gripping regions (52) may be reconfigured or modified, if not omitted altogether, as desired.

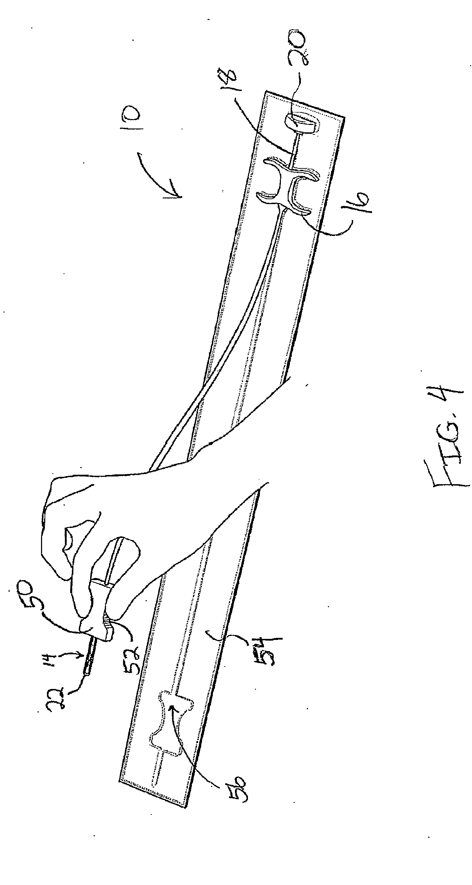

As is also shown, slider (50) is initially disposed near the distal end of marker applier (10), though proximal to opening (14). This initial position of slider (50) may be substantially fixed by a packaging tray (54). In particular, as shown in FIG. 4, packaging tray (54) may include a recessed region (56) that is configured to releasably engage marker applier (10) and slider (50). Of course, packaging tray (54) and recessed region (56) are merely exemplary, and these items may be modified, supplemented, or omitted as desired.

In some versions, cannula (12) has a significant length (e.g., up to approximately 18" or more) and flexibility that, if marker applier (10) is held only by grip (16) without any additional support (e.g., without any support in the distal region of cannula (12)), cannula (12) may significantly droop under its own weight. Such drooping may occur even in the absence of slider (50) in some configurations of cannula (12), and may make it difficult to feed tip (22) into an opening without grasping some distal portion of cannula (12). Alternatively, the length of cannula (12) and/or the materials or other properties of cannula (12) may be such that no significant drooping occurs. Similarly, drooping of cannula (12) may be irrelevant in some contexts.

As shown in FIG. 4, when removing marker applier (10) from packaging (54), or when otherwise handling marker applier (10). A user may grasp marker applier (10) by gripping slider (50). In particular, gripping regions (52) may be configured for a user to grasp slider (50) at gripping regions (52). Furthermore, gripping regions (52) may be knurled, include ridges or protuberances, or have other structural features or properties to facilitate gripping. In addition to grasping marker applier by slider (50), a user may grasp grip (16) with the user's other hand. Alternatively, the user may simply grasp marker applier (10) by slider (50) alone.

In some contexts, by gripping maker applier (10) by slider (50), the user may avoid contacting cannula (12) directly with the user's hands. In some contexts, this avoidance of direct contact with cannula (12) may reduce the likelihood of the user contaminating an otherwise sterilized cannula (12). Furthermore, to the extent that cannula (12) may otherwise droop, a user may avoid drooping of cannula (12) by grasping marker applier (10) by slider (50).

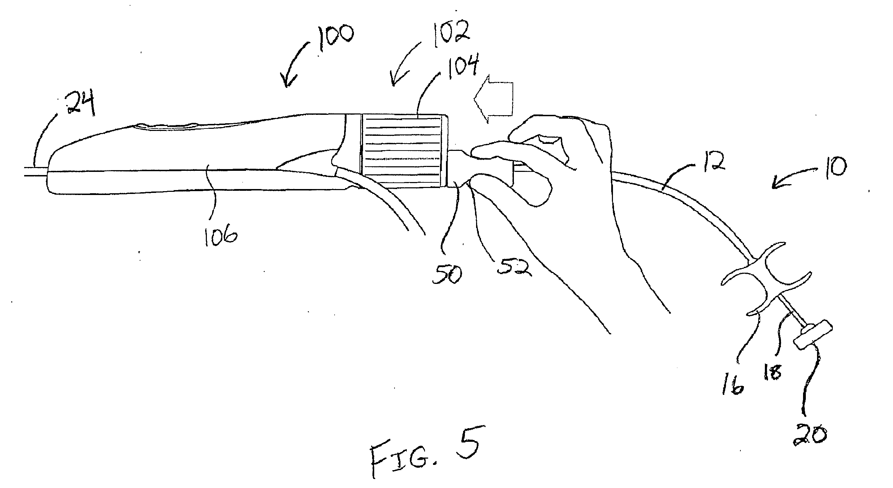

As shown in FIGS. 5-6, slider (50) may also be employed when engaging marker applier (10) with a biopsy device (100). For instance, and as noted above, biopsy device (100) may include any of the various biopsy devices disclosed in U.S. Non-Provisional Patent Application Serial No. 11/942,785, entitled "Revolving Tissue Sample Holder for Biopsy Device," filed November 21, 2007, the disclosure of which is incorporated by reference herein. In the present example, biopsy device (100) includes a tissue sample holder (102) that has a rotatable member for indexing a plurality of discrete tissue sample compartments to successively collect a plurality of tissue samples. Tissue sample holder (102) has an outer cup (104) in this example. A passage (not shown) is formed through outer cup (104) and tissue sample holder (102) for inserting cannula (12) of marker applier (10) therethrough, to pass a portion of cannula (12) into needle (24) of biopsy device (100) as described above.

As shown in FIG. 5, the user may use slider (50) to facilitate insertion of tip (22) in the passage formed through outer cup (104) and tissue sample holder (102). In other words, use of slider (50) may make it easier for the user to insert tip (22) in biopsy device (100) than it would be to accomplish the same by only grasping grip (16), by reducing the distance between the user's fingertips and the insertion point.

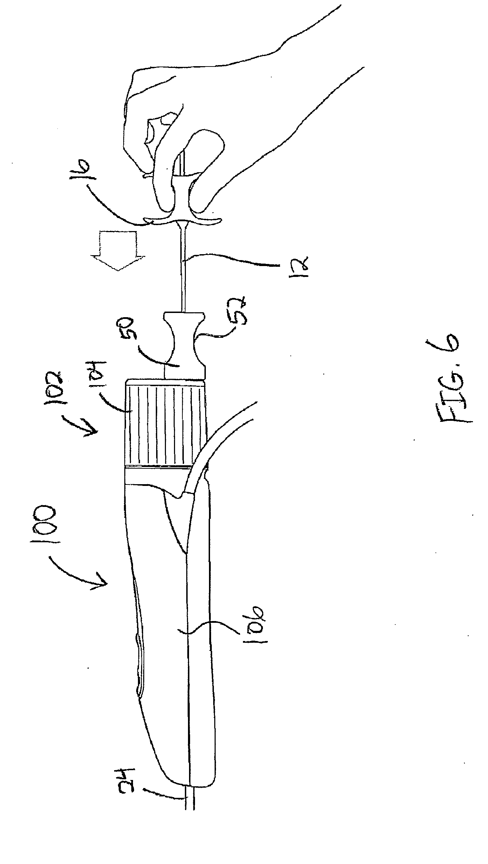

Outer cup (104) and/or slider (50) may also include one or more features for selectively securing slider (50) to outer cup (104) (or for selectively securing slider (50) relative to outer cup (104)). For instance, a portion of slider (50) may "snap" into cup (104). Engagement between slider (50) and cup (104) may alternatively require some degree of rotation of slider relative to cup (104). Alternatively, other forms of engagement may be used, or slider (50) may be configured such that it does not secure to cup (104) or some other component of biopsy device (100). As shown in FIG. 6, after slider (50) has been positioned and secured relative to outer cup (104) in this example, the user may feed cannula (12) distally through biopsy device (100), such as by grasping grip (16).

Slider (50) may be configured such that cannula (12) may slide freely through slider (50) as the user feeds cannula (12) distally through biopsy device (100). In some versions, slider (50) may be configured such that the user must actuate a button, lever, release, or other structure on slider (50) in order for cannula (12) to slide freely through slider (50). Slider (50) may include a feature that is operable to selectively lock and/or unlock the longitudinal position of slider (50) along cannula (12). Alternatively, slider (50) may be configured such that slider (50) always slides freely along cannula (12). Of course, any other features or configurations may be used. As noted above, there are a variety of other ways in which marker applier (10) and slider (50) may be used and operated. Furthermore, there are a variety of ways in which marker applier (10) and slider (50) may be modified. Several such modifications will be described in greater detail below, while other modifications will be apparent to those of ordinary skill in the art in view of the teachings herein.

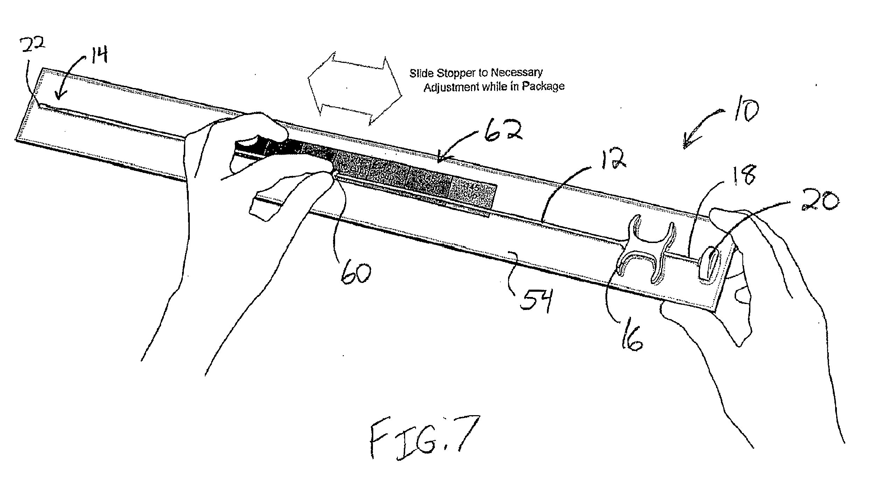

One merely exemplary variation of marker applier (10) is shown in FIG. 7. As shown, maker applier (10) includes a sliding stopper (60) disposed about cannula (12). In this example the relationship between stopper (60) and cannula (12) is such that the longitudinal position of stopper (60) about cannula (12) is substantially fixed until a user moves stopper (60) with sufficient force. For instance, stopper (60) may be formed of a resilient member that is configured to grip cannula (12).

As is also shown in FIG. 7, packaging (54) may include one or more visual indicators (62) associated with an insertion depth for cannula (12). Such indicators (62) may include color-coded regions, numerical descriptions, textual descriptions, graphical indications, and/or may have any other suitable characteristics. For instance, indicators (62) may numerically indicate an insertion depth for cannula (12). Indicators (62) may also include textual description relating to selectable insertion depths, such as description relating to whether the insertion depth factors in a cup (104) being secured to a biopsy device (100) or not (e.g., depth varies based on presence of cup (104) or other component of biopsy device (100)). The user may then slide stopper (60) to a position within a region (or at a point) designated by an indicator (62) associated with a desired depth of insertion.

The depth of insertion may relate to the longitudinal distance between the tip (22) and stopper (60) and/or the longitudinal distance between the opening (14) and stopper (60), among other things. By way of example only, marker applier (10) may be configured to be used in conjunction with a variety of biopsy devices (100). Such biopsy devices (100) may have a variety of different characteristics that may influence the depth of insertion of a marker applier (10), including but not limited to the length of needle (24), length of handpiece (106), length or presence of tissue sample holder (102), length or presence of outer cup (104), and/or a variety of other characteristics. The presence of stopper (60) and/or indicators (62) may thus facilitate use of a marker applier (10) with a variety of biopsy devices (100). For instance, the marker applier (10) can include multiple indicators, including indicators corresponding to different biopsy devices.

It should be understood that, in addition to or in lieu of indicators (62) on packaging (54), indicators (62) may be provided on cannula (12). Such indicators (62) on a cannula (12) may have any desired similarities or differences relative to indicators (62) on packaging (54). Alternatively, cannula (12) and/or packaging (54) may lack indicators (62).

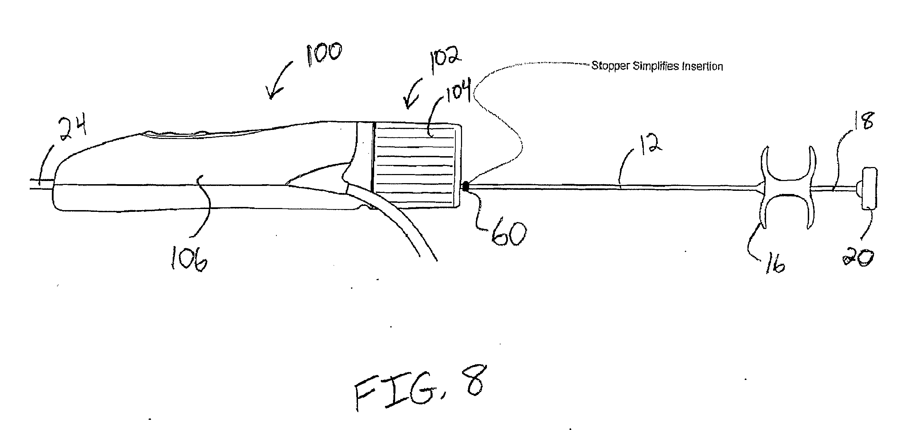

As shown in FIG. 8, stopper (60) may be configured to engage cup (104) or some other component of biopsy device (100). In particular, stopper (60) may be configured to substantially restrict the length to which cannula (12) may be inserted in biopsy device (100). For instance, stopper (60) may present an outer diameter that exceeds the inner diameter of an opening in cup (104). Other ways in which stopper (60) may restrict distal insertion of cannula (12) into biopsy device (100) will be apparent to those of ordinary skill in the art in view of the teachings herein.

As also noted above, the relationship between stopper (60) and cannula (12) may be such that stopper (60) presents some significant degree of resistance to longitudinal movement of stopper (60) along cannula (12). For instance, such resistance may be low enough to permit a user to slide stopper (60) to a desired longitudinal position along cannula (12); yet high enough to prevent stopper (60) from moving along cannula (12) to some degree when the user inserts cannula (12) in biopsy device (100) in a normal use. In other words, stopper (60) may provide the user with tactile feedback indicating engagement between stopper (60) and the biopsy device (100) as the user is inserting cannula (12) into biopsy device (100). Such tactile feedback may thus indicate to the user that cannula (12) has been inserted to the desired depth. Suitable structures, materials, configurations, relationships, and techniques for providing such resistance or tactile feedback will be apparent to those of ordinary skill in the art in view of the teachings herein.

It will also be appreciated that features, functions, and/or operational principles of slider (50) and stopper (60) may be combined in a variety of ways. For instance, slider (50) may provide some degree of resistance to longitudinal movement along cannula (12) as described in the context of stopper (60) above. Similarly, packaging (54) associated with a marker applier (10) having a slider (50) may have indicators (62) similar to those described above with respect to stopper (60). Alternatively, features, functions, and/or operational principles of slider (50) and stopper (60) may be combined in any other suitable fashion.

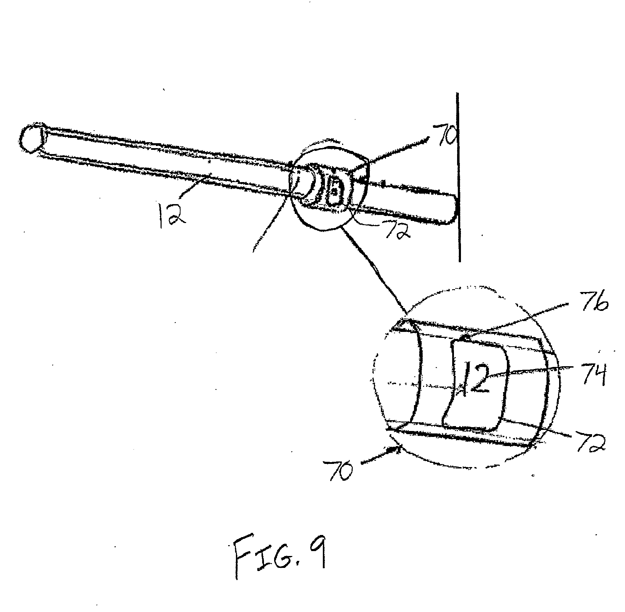

Another merely exemplary variation of marker applier (10) is shown in FIG. 9, in which cannula (12) of marker applier (10) is shown but certain other components are omitted. As shown, maker applier (10) includes a slider (70) disposed about cannula (12). Slider (70) may have a ring-like configuration or other configuration. Slider (70) of this example includes a window (72). In some versions, window (72) includes an enlarging lens formed of any suitable material (e.g., plastic, glass, etc.). In addition, cannula (12) may include one or more indicators (74). Indicators (74) may have any desired similarities or differences relative to indicators (62) described above in the context of packaging (54). Slider (70) may thus be moved longitudinally along cannula (12) until window (72) is positioned over an indicator (74) that indicates a desired depth of insertion. To the extent that window (72) includes a lens, such a lens may facilitate viewing of the indicator (74).

One or more detents (76) may also be included. For instance, each indicator (74) may have a detent (76) associated with it. Such a detent (76) may provide tactile feedback to a user as the user moves slider (70) along cannula (12), indicating that window (72) is passing over an indicator (74). Detents (76) may also provide some degree of resistance to longitudinal movement of slider (70) along cannula (12), such that detents (76) may reduce the likelihood that slider (70) will be inadvertently moved from a selected indicator (74) during insertion of cannula (12) in biopsy device (100). Alternatively, any suitable structures other than detents (76) may be used, if anything at all.

Slider (70) may also be configured to engage biopsy device (100) (e.g., by engaging cup (104)) as cannula (12) is inserted in biopsy device (100). Slider (70) may thus facilitate insertion of cannula (12) to a desired depth, similar to stopper (60) described above. Furthermore, as with stopper (60) and slider (50), it should be understood that features, functions, and/or operational principles of slider (70) may be interchanged among slider (50), stopper (60), and slider (70). For instance, slider (50) may be modified to include a window (72) in addition to one or more features for providing some appreciable degree of resistance to longitudinal movement along cannula (12). Other ways in which features, functions, and/or operational principles of slider (50), stopper (60), and slider (70) may be interchanged will be apparent to those of ordinary skill in the art in view of the teachings herein.

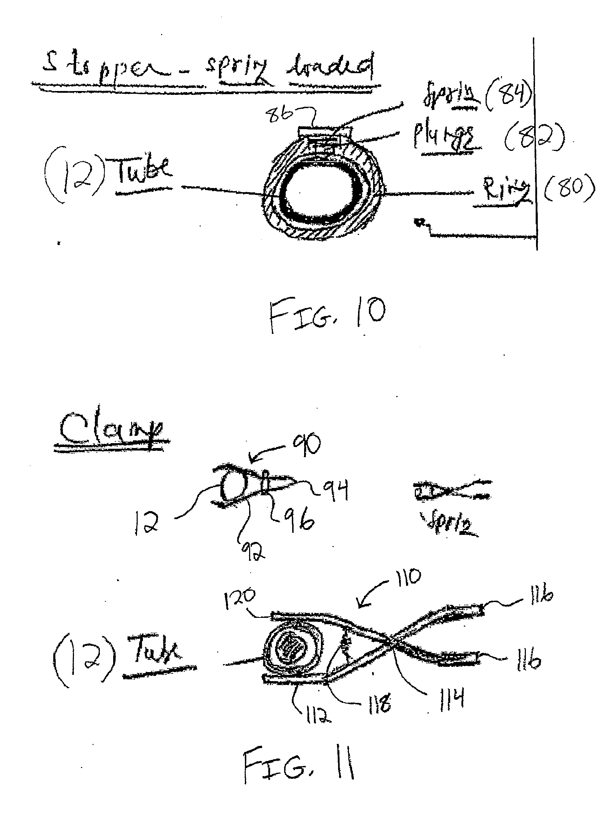

Yet another variation of marker applier (10) is shown in FIG. 10, in which a cross section of cannula (12) of marker applier (10) is shown but certain other components are omitted. As shown, maker applier (10) includes a stopper (80) disposed about cannula (12). Stopper (80) may have a ring-like configuration or other configuration. Stopper (80) of this example includes a detent feature (82) that is loaded with a spring (84). Detent feature (82) may be used to selectively lock the longitudinal position of stopper (80) along cannula (12). In particular, spring (84) may be configured to bias detent feature (82) against cannula (12). In some versions, a button (86) or other feature is provided. The user may depress such a button (86) or otherwise engage a feature to selectively disengage the detent feature (82) from the cannula (12). Upon release of button (86), spring (84) may re-engage detent feature (82) with cannula (12).

Of course, a variety of other features, spring-loaded or otherwise, may be used to selectively engage a stopper (80) with a cannula (12), in addition to or in lieu of detent feature (82). Furthermore, any features, functions, and/or operational principles of stopper (80) may be interchanged among slider (50), stopper (60), slider (70), and stopper (80) as desired.

Still other variations of marker applier (10) are shown in FIG. 11, in which cross sections of cannula (12) of marker applier (10) is shown but certain other components are omitted. As shown, maker applier (10) includes a clamp (90, 110) disposed about cannula (12). One version of clamp (90) comprises a pair of arms (92) that are joined at a common point (94). In particular, arms (92) are configured to pivot relative to one another about point (94). Clamp (90) may thus be configured similar to tweezers or calipers. Clamp (90) may also include a locking member (96) that is configured to secure the pivotal position of arms (92) relative one another. Locking member (96) may also be actuated to draw arms (92) pivotally toward one another. For instance, arms (92) may be pivotally relaxed to permit clamp (90) to be positioned at a desired longitudinal position along cannula (12), and locking member (96) may then be actuated to draw arms (92) together and secure their position. Arms (92) may thereby secure clamp (90) to cannula (12) by clamping down on it. Suitable features and components for a locking member (96) will be apparent to those of ordinary skill in the art in view of the teachings herein.

Clamp (110) also comprises a pair of arms (112). Arms (112) cross each other and are pivotally connected at a hinge (114). A resilient member (118) (e.g., a spring, etc.) is biased to pull first ends (120) of arms (12) toward one another. In other versions, resilient member (118) is positioned on the other side of hinge (114), and is biased to push first ends (120) toward one another. Clamp (110) may be operated by a user pushing second ends (116) of arms (12) toward one another. In particular, a user may push second ends (116) toward one another to create a gap between first ends (120) that is greater than the diameter of a cannula (12). The user may then position the clamp (110) at a desired location along the length of cannula (12). Then the user may release second ends (116), such that resilient member (118) may draw first ends (120) toward one another to clamp down on cannula.

Like stopper (80), clamps (90, 110) may be secured to cannula (12) to limit the depth to which cannula (12) may be inserted in a biopsy device (100). It will be appreciated that clamps (90, 110) may be varied in a number of ways. It will also be appreciated that any features, functions, and/or operational principles of clamps (90, 110) may be interchanged among slider (50), stopper (60), slider (70), stopper (80), and clamps (90, 110) as desired. For instance, a slider (50) may include a clamping feature similar to any one of clamps (90, 110), as well as a window (72) with a magnifying lens to view indicators (74) on a cannula (12). Still other suitable combinations and interchanges among slider (50), stopper (60), slider (70), stopper (80), and clamps (90, 110), as well as additional variations of cannula (12) and other components of marker applier (10), will be apparent to those of ordinary skill in the art in view of the teachings herein.

In some other versions, sliders (50, 70) or stoppers (60, 80) are configured to selectively engage cannula (12) upon a certain degree of relative rotation between sliders (50, 70) or stoppers (60, 80) and cannula (12). For instance, sliders (50, 70) or stoppers (60, 80) and cannula (12) may have cross-sectional shapes or surface features that permit sliders (50, 70) or stoppers (60, 80) to move longitudinally along cannula (12) when sliders (50, 70) or stoppers (60, 80) are at a first rotational position about cannula (12); yet restrict or prevent longitudinal movement of sliders (50, 70) or stoppers (60, 80) along cannula (12) when sliders (50, 70) or stoppers (60, 80) are at a second rotational position about cannula (12). Suitable examples of such rotational lock shapes and features are disclosed in U.S. Pub. No. 2007/0255168, entitled "Grid and Rotatable Cube Guide Localization Fixture for Biopsy Device," published November 2, 2007, the disclosure of which is incorporated by reference herein; and U.S. Pub. No. 2007/0255170, entitled "Biopsy Cannula Adjustable Depth Stop," published November 1, 2007, the disclosure of which is incorporated by reference herein.

Embodiments of the present invention may have application in conventional endoscopic and open surgical instrumentation as well as application in robotic-assisted surgery.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before surgery. First, a new or used instrument may be obtained and if necessary cleaned. The instrument may then be sterilized. In one sterilization technique, the instrument is placed in a closed an sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

### Embodiments of the invention not yet claimed:

1. An apparatus for delivering biopsy markers to a biopsy site, the apparatus comprising:
   a cannula having a lumen extending at least partially therethrough, and a marker exit opening communicating with the lumen;
   at least one biopsy marker disposed in the lumen for deployment through the marker exit opening in the cannula; and
   a movable member positioned on the cannula, wherein the position of the member along the length of the cannula is adjustable.
2. The apparatus of embodiment 1 wherein the movable member is adapted to slide axially with respect to the cannula.
3. The apparatus of embodiment 1 further comprising a grip associated with a proximal end of the cannula, and wherein the movable member is positionable along the length of the cannula between the grip and a distal tip of the cannula.
4. The apparatus of embodiment 1 further comprising an indicator operatively associated with the movable member for indicating the position of the movable member with respect to a reference position.
5. The apparatus of embodiment 4 wherein the indicator comprises a visual indicator.
6. The apparatus of embodiment 4 wherein the indicator provides information with respect to a depth of insertion of a portion of the cannula.
7. The apparatus of embodiment 4 wherein the indicator comprises a tactile indicator.
8. The apparatus of embodiment 1, wherein apparatus is packaged with the movable member in a predetermined position along the length of the cannula.
9. The apparatus of embodiment 1 wherein the apparatus is disposed in a package, and
   wherein the apparatus is capable by being removed from the package by grasping the movable member.
10. The apparatus of embodiment 1 further comprising a feature for releasably locking the movable member at a plurality of positions along the length of the cannula.
11. A biopsy system comprising:
   a biopsy device operable for taking one or more biopsy samples from a patient, the biopsy device comprising a piercing member and cutter for severing tissue received within the piercing member;
   a biopsy marker applier, the marker applier comprising a cannula having a marker lumen and at least one marker disposed in the marker lumen, and a movable member positionable on the cannula;wherein the movable member is positionable on the cannula at a plurality of locations along the length of the cannula.
12. The system of embodiment 11 wherein the movable member is positionable on the cannula to provide a desired insertion of a portion of the biopsy marker applier into the biopsy device or a patient.
13. The system of embodiment 11 further comprising an indicator for providing an indication of a depth of insertion of a portion of the biopsy marker applier into the biopsy device or a patient.
14. A method of delivering a biopsy marker to a biopsy site, the method comprising the steps of:
   obtaining a biopsy marker applier comprising at least one biopsy marker;
   determining a desired depth of insertion of a portion of the biopsy marker applier within a biopsy device or a patient; and
   positioning a movable member along a portion of the biopsy marker applier to a position corresponding to the desired depth of insertion.
15. The method of embodiment 14 wherein the step of positioning comprises sliding the movable member along an axial length of a portion of the biopsy marker applier.

## Claims

1. An apparatus (10) for delivering a biopsy marker to a biopsy site, the apparatus comprising:
a cannula (12) having a lumen, a closed distal end, a lateral marker exit opening (14) disposed proximally of the closed distal end;
at least one biopsy marker (30) disposed in the lumen for lateral deployment through the lateral marker exit (14);
a movable member slidably disposed on an outer surface of the cannula, wherein
the position of the movable member on the cannula is adjustable for positioning the lateral marker exit opening (14) with respect to a reference position.

2. The apparatus of Claim 1 wherein the cannula is flexible.

3. The apparatus of Claim 1 comprising a ramp positioned with respect to the lateral marker exit opening, the ramp being operable to direct the at least one marker through the lateral marker exit opening.

4. The apparatus of Claim 1 further comprising an indicator operatively associated with the movable member for indicating the position of the movable member with respect to a reference position.

5. A biopsy system comprising:
a biopsy device (100) operable for taking one or more biopsy samples, the biopsy device including a needle extending distally from the biopsy device and a tissue sample holder disposed at a proximal end of the biopsy device,
wherein the tissue sample holder is adapted to receive biopsy samples transported through the needle;
a biopsy marker delivery apparatus having a cannula with a closed distal end and a lateral marker exit opening;
wherein a distal portion of the cannula of the biopsy marker delivery apparatus is operative to be inserted through the tissue sample holder and into the needle of the biopsy device;
and wherein the biopsy marker delivery apparatus has a slidable member disposed on an outer surface of the biopsy marker delivery cannula, the slidable member sized so as to not be insertable through the tissue sample holder.
